# FASCICULE DE BREVET EUROPEEN

(11) **EP 1 363 995 B1**
(45) Date de publication et mention de la délivrance du brevet: **20.01.2010**
(21) Numéro de dépôt: 01976371.3
(22) Date de dépôt: 05.10.2001
(51) Int. Cl.: C12N 5/10, C12N 15/66, C12N 1/38, A61K 48/00, C12N 15/86

(54) **UTILISATION D'UNE COMPOSITION COMPRENANT DE LA N-ACÉTYL-CYSTÉINE POUR LE CONDITIONNEMENT DE CELLULES SOUCHES**
VERWENDUNG EINER ZUSAMMENSETZUNG MIT N-ACETYLCYSTEIN ZUR KONDITIONIERUNG VON STAMMZELLEN
USE OF A COMPOSITION COMPRISING N-ACETYLCYSTEINE FOR CONDITIONING STEM CELLS

(30) Priorité: 06.10.2000 FR 0012845
(43) Date de publication de la demande: 26.11.2003
(73) Titulaire: IGR ET D, F-94800 Villejuif Cedex (FR); Cohen-Haguenauer, Odile, F-75116 Paris (FR)
(72) Inventeur: COHEN-HAGUENAUER, Odile, F-75116 Paris (FR)
(74) Mandataire: Breese, Pierre
(86) Numéro de dépôt international: PCT/FR2001/003087
(87) Numéro de publication internationale: WO 2002/029011

(56) Documents cités:
- EP-A- 0 839 534
- WO-A1-99/56759
- WO-A2-00/05343
- MORSHEAD CINDI M ET AL: "A cell-survival factor (N-acetyl-L-cysteine) alters the in vivo fate of constitutively proliferating subependymal cells in the adult forebrain." JOURNAL OF NEUROBIOLOGY, vol. 42, no. 3, 15 février 2000 (2000-02-15), pages 338-346, XP001011897 ISSN: 0022-3034
- LUCCA ELISA ET AL: "Influence of cell culture conditions on the protective effect of antioxidants against beta-amyloid toxicity: Studies with lazaroids." BRAIN RESEARCH, vol. 764, no. 1-2, 1997, pages 293-298, XP001011311 ISSN: 0006-8993
- LADEROUTE KEITH R ET AL: "Hypoxia/reoxygenation stimulates jun kinase activity through redox signaling in cardiac myocytes." CIRCULATION RESEARCH, vol. 80, no. 3, 1997, pages 336-344, XP001011882 ISSN: 0009-7330
- FU KAI-LING ET AL: "Functional correction of Fanconi anemia group A hematopoietic cells by retroviral gene transfer." BLOOD, vol. 90, no. 9, 1997, pages 3296-3303, XP002172368 ISSN: 0006-4971 cité dans la demande
- FREIE B W ET AL: "CORRECTION OF FANCONI ANEMIA TYPE C PHENOTYPIC ABNORMALITIES USING A CLINICALLY SUITABLE RETROVIRAL VECTOR INFECTION PROTOCOL" CELL TRANSPLANTATION,US,ELSEVIER SCIENCE, vol. 5, no. 3, 1996, pages 385-393, XP000867303 ISSN: 0963-6897
- COHEN-HAGUENAUER O ET AL: "TRANSDUCTION OF HUMAN CD34+ HAEMOPOIETIC PROGENITORS OF VARIOUS ORIGIN USING AN ORIGINAL RETROVIRUS VECTOR DERIVED FROM FR-MULV AND CLINICALLY RELEVANT PROCEDURES" GENE THERAPY,GB,MACMILLAN PRESS LTD., BASINGSTOKE, vol. 2, no. SUPPL. 01, 1 novembre 1995 (1995-11-01), page S19 XP000617113 ISSN: 0969-7128
- PIACIBELLO WANDA ET AL: "Engraftment in nonobese diabetic severe combined immunodeficient mice of human CD34+ cord blood cells after ex vivo expansion: Evidence for the amplification and self-renewal of repopulating stem cells." BLOOD, vol. 93, no. 11, 1 juin 1999 (1999-06-01), pages 3736-3749, XP002172369 ISSN: 0006-4971
- TRAYCOFF C M ET AL: "PROLIFERATION-INDUCED DECLINE OF PRIMITIVE HEMATOPOIETIC PROGENITORCELL ACTIVITY IS COUPLED WITH AN INCREASE IN APOPTOSIS OF EX VIVO EXPANDED CD34+ CELLS" EXPERIMENTAL HEMATOLOGY, NEW YORK, NY, US, vol. 26, no. 1, 1998, pages 53-62, XP001002880 ISSN: 0301-472X
- DINSMORE ET AL: 'Embryonic stem cells differentiated in vitro as a novel source of cells for transplantation' CELL TRANSPLANTATION vol. 5, no. 2, 1996, pages 141 - 143, XP002920536
- WOBUS A.M. ET AL: 'Retinoic acid accelerates embryonic stem cell-derived cardiac differentiation and enhances development of ventricular cardiomyocytes' J. MOL. CELL CARDIOL. vol. 29, 1997, pages 1525 - 1539, XP001030714
- KEIL F. ET AL: 'Effect of interleukin-3, stem cell factor and granulocyte-macrophage colony-stimulating factor on comitted stem cells, long-term culture initiating cells and bone marrow stroma in one-step, long-term bone marrow culture' ANNALS OF HEMATOLOGY vol. 79, 2000, pages 243 - 248
- VAN BOCKSTAELE D.R. ET AL: 'Direct efefcts of 13-cis and all-trans retinoic acid on normal bone marrow (BM) progenitors: comparative study on BM mononuclear cells and on isolated CD34+ BM cells' ANNALS OF HEMATOLOGY vol. 66, no. 2, 01 Janvier 1993, BERLIN, DE, pages 61 - 66, XP008064483
- DATABASE BIOSIS [en ligne] BIOSCIENCES INFORMATION SERVICE, PHILADELPHIA, PA, US PREV200100322038 & LIU ET AL: "Apoptosis and poor proliferation in the absence of stroma is responsible for poor retroviral gene transfer in CD34+ progenitors from Fanconi anemia" BLOOD, vol. 96, no. 11-1, novembre 2000 (2000-11), page 230A,
- COHEN-HAGENAUER O. ET AL: 'In vivo repopulation ability of genetically corrected bone marrow cells from Fanconi anemia patients' PNAS vol. 103, no. 7, 2006, pages 2340 - 2345
- ROSENZWEIG M. ET AL: 'Efficient and durable gene marking of hematopoeitic progenitor cells in nonhuman primates after nonablative conditioning' BLOOD vol. 94, no. 7, 1999, pages 2271 - 2286, XP002175410

## Description

La présente demande a pour objet une nouvelle utilisation de compositions à activité antioxydante, comprenant de la N-acétylcystéine pour le conditionnement de cellules souches autres que des cellules souches embryonnaires humaines.

Les cellules souches sont des cellules appartenant à des répertoires diversifiés, ces cellules étant douées de capacités d'auto-renouvellement et, plus précisément, pouvant être totipotentes, et de ce fait capables de donner naissance à un organisme entier, ou pluripotentes c'est à dire capables d'engendrer toutes les cellules de l'organisme à l'exception des trophoblastes placentaires - ces dernières sont par exemple les cellules souches embryonnaires (ES) qui semblent pouvoir être maintenues in vitro dans un état indifférencié de façon quasi-indéfinie - ou encore multipotentes, c'est à dire capables d'engendrer perpétuellement les cellules d'une lignée donnée.

En s'auto-renouvelant, les cellules souches donnent, pour une partie d'entre elles, des cellules filles dont le potentiel souche est maintenu et, pour une autre partie, des cellules filles capables de proliférer pour conduire à des cellules différenciées.

Les cellules souches ont en général des intermédiaires dans la voie de la différenciation, des progéniteurs, dont la capacité de prolifération est limitée et le potentiel de différenciation restreint.

Cependant, si leur utilisation est large à titre de modèle expérimental chez la souris, la mise en oeuvre de protocoles expérimentaux avec des cellules d'origine humaine connaît à l'heure actuelle d'importantes restrictions d'ordre éthique et légal toutefois variable d'un pays à l'autre. L'exploitation des lignées est factuelle.

On désigne par le terme « prolifération », conformément à la définition habituellement retenue, la multiplication des cellules *in vitro,* qui s'accompagne, dans le cas spécifique des cellules souches, nécessairement d'une phase de différenciation (à la différence de leur simple amplification). En effet, les cellules souches siègent au sommet de la hiérarchie d'une lignée et prolifèrent in vitro en formant les cellules différenciées d'un tissu donné.

Avantageusement, la prolifération cellulaire peut alternativement, ou éventuellement également, être constatée *in vivo.*

La capacité de prolifération peut donc être considérée comme une caractéristique intrinsèque des cellules souches, en ce que le processus de prolifération et de différenciation représente le devenir normal de telles cellules. Aussi est-ce à cette capacité de prolifération qu'il sera fait référence dans la suite de la présente demande à travers l'expression « potentiel souche ».

Dans le cas de la présente invention, il convient de considérer que la prolifération des cellules souches impose une étape indissociable de différenciation de ces cellules. Inversement, le processus de différenciation des cellules souches s'entend comme s'inscrivant nécessairement dans le cadre d'une phase de prolifération préalable desdites cellules.

Les cellules souches des organismes adultes représentent également un potentiel tout à fait important dans la perspective de leur utilisation thérapeutique. A cet égard, les moyens décrits dans le cadre de l'invention peuvent être appliqués:
- aux cellules souches du système nerveux central, capables de se différencier en cellules souches hématopoiétiques, neurones, astrocytes, oligodendrocytes,
- aux cellules souches hématopoiétiques, en particulier aux hémangioblastes. Les cellules souches hématopoiétiques sont des cellules ayant in vivo, une capacité de reconstitution hématologique,
- aux cellules souches mésenchymateuses, capables de se différencier vers des entités variées incluant l'os, le cartilage, la graisse, les tendons, les muscles et le stroma médullaire, pour notamment se différencier en adipocytes, chondrocytes ou ostéocytes,
- les cellules souches musculaires,
- les cellules souches musculaires ou hépatiques ou cérébrales, obtenues à partir de cellules de la moelle osseuse.

Toutes ces cellules souches constituent des cellules cibles intéressantes, notamment pour le transfert de gènes, dans le cadre de protocoles de thérapie génique. Cependant le transfert efficace de séquences nucléotidiques d'intérêt (désignées par le terme "gène" dans la suite) pose différents problèmes dont certains sont liés à la nécessité de maintenir ces cellules en culture, pendant un temps suffisant pour les manipuler en vue de leur utilisation ultérieure, et notamment dans des conditions permettant ultérieurement le transfert de gènes de façon efficace, afin en particulier d'envisager leur implantation chez des patients. Les difficultés se trouvent encore accrues par le fait que ce temps de maintien en culture, bien que nécessaire, ne doit en aucun cas entraîner la perte du potentiel souche, qui est en l'espèce la caractéristique recherchée justifiant l'utilisation de cellules souches. En d'autres termes, les cellules souches, malgré le temps de culture nécessité par les protocoles de thérapie génique, ne doivent pas s'inscrire dans un programme irréversible de différenciation terminale.

Ces cellules souches sont aussi des cibles intéressantes pour le maintien en culture des cellules, éventuellement de leur amplification et/ou de leur prolifération et/ou de leur différenciation. En particulier, ces cellules souches sont des cibles intéressantes pour le maintien en culture tout en préservant leur potentiel souche ; que la destinée des cellules soit l'amplification et/ou la prolifération est égal.

On entend par le terme « amplification », conformément à la définition connue, la faculté, au sein d'un compartiment cellulaire, par exemple pour un contingent de cellules souches, d'augmenter en nombre, tout en conservant leurs caractéristiques intrinsèques, et cela, sans qu'aucun processus de différenciation terminale ne soit mis en jeu immédiatement. En particulier, dans les situations qui impliquent une seule voie de différenciation des cellules souches, la fonction essentielle de la population intermédiaire de progéniteurs consiste précisément en une amplification, dont l'effet est d'accroître ensuite le nombre de cellules différenciées provenant de chaque division d'une cellule souche.

La présente demande met en évidence que les cellules souches, autres que des cellules souches embryonnaires humaines et en particulier les cellules souches décrites plus haut, peuvent être conditionnées de façon à permettre leur culture pendant des durées suffisantes pour envisager le transfert de gènes d'intérêt, et/ou pour permettre leur auto-renouvellement et/ou leur amplification et/ou leur différenciation et/ou leur prolifération et/ou la régénération du potentiel souche.

Il est entendu, dans le cadre de la présente invention, que chaque formé de conditionnement des cellules souches définie ci-dessus présente, en tant que telle, un intérêt spécifique. Entre également dans le cadre de l'invention, la combinaison des états desdites cellules résultant du conditionnement selon l'invention et, à titre d'exemple, le conditionnement permet le maintien en culture et l'amplification, ou le maintien en culture et la différenciation, ou l'amplification et la différenciation. Selon un mode de réalisation particulier de l'invention, le conditionnement permet la prolifération des cellules traitées.

En outre les moyens définis dans le cadre de l'invention peuvent conférer aux cellules conditionnées incorporant lesdits gènes par transformation, un avantage sélectif qui se manifeste i) au niveau du nombre de colonies cellulaires formées (CFU), témoignant d'une capacité de prolifération, et/ou ii) au niveau de la qualité (taille, nature) des cellules en culture, et/ou (iii) par une régénération du potentiel souche lorsqu'il est affecté de façon pathologique.

L'invention définit en particulier des conditions de culture des cellules souches favorables à la transduction efficace de ces cellules par des vecteurs portant des gènes d'intérêt, par exemple des vecteurs rétroviraux, des vecteurs lentiviraux, des vecteurs tels que SV40, ou tous vecteurs susceptibles de transformer ou de transduire des cellules souches.

L'invention a donc pour objet, l'utilisation d'une composition à activité antioxydante, pour le conditionnement in vitro de cellules souches autres que des cellules souches embryonnaires humaines.

L'expression "composition à activité antioxydante" définit des substances, utilisées seules ou en mélange, qui ont des propriétés antioxydantes au niveau des cellules eucaryotes. On observera que dans le cadre de l'invention, l'activité antioxydante est un critère de sélection des compositions utilisées, mais que les propriétés antioxydantes de ces compositions ne sont pas nécessairement les seules mises en oeuvre.

Selon le mode de réalisation de l'invention, la composition utilisable pour le conditionnement des cellules souches, comprend la N-acétylcystéine.

L'article de C. Morshead et D. van der Kooy publié dans Journal of Neurobiology, 42: 338-346 (1999), rapporte les effets protecteurs anti-oxydants de la N-acétyl-cystéine sur un culture des cellules corticales dans un milieu dépourvu de sérum.

L'article de E. Lucca et al publié dans Brain Research, 764: 293-298 (1997) décrit l'infusion de N-acétyl-cystéine dans le ventricule latéral du cerveau d'une souris adulte. L'infusion intraventriculaire favorise la survie des cellules subependymales in vivo.

Dans le cadre de l'invention, la composition à activité antioxydante est utilisée pour le conditionnement des cellules souches dans des conditions permettant le maintien en culture de ces cellules, leur auto-renouvellement et/ou leur amplification et/ou leur prolifération et/ou leur différenciation et/ou la régénération de leur potentiel souche.

Le maintien en culture des cellules souches conditionnées selon l'invention doit être assuré pendant une durée suffisamment longue pour envisager leur utilisation en vue de l'implantation chez un patient, le cas échéant après transfert d'un gène d'intérêt.

Selon un mode de réalisation particulier de l'invention, la qualité des cellules conditionnées est également améliorée, de sorte qu'après transfert de gène, ces cellules présentent un avantage sélectif vis à vis des cellules non transformées. En particulier les inventeurs ont observé que les cellules souches hématopoiétiques conditionnées selon l'invention, transduites avec un vecteur portant un gène correcteur du gène déficient mis en évidence dans la maladie de Fanconi, montrent un avantage sélectif en culture, par rapport aux cellules non transduites.

Les inventeurs ont en outre constaté que les cellules souches hématopoiétiques ainsi conditionnées, puis transduites au moyen de vecteurs portant un gène d'intérêt, peuvent également présenter une amélioration qualitative en taille et en nature (présence constatée de BFU-E et CFU-GEMM), par rapport à des cellules non transduites.

A titre d'exemple, des cellules souches conditionnées selon l'invention peuvent avantageusement être maintenues en culture pendant une durée d'au moins 2 jours, de préférence d'environ 7 jours correspondant au temps de faisabilité clinique, et pour des besoins d'évaluation, pendant une durée d'au moins 30 jours, de préférence d'au moins 35 jours après transfert de gène. Les cellules transduites peuvent être évaluées à j35 après la mise en culture, en vue de leur utilisation ultérieure. On constate que ces cellules conditionnées avec la composition à activité antioxydante puis génétiquement manipulées sont capables de surmonter le blocage du cycle cellulaire induit par un traitement à la Mitomycine C (MMC) (10 nM-pendant 7 jours puis 100mM pendant 48heures).

De manière tout à fait surprenante, les cellules souches conditionnées selon l'invention peuvent être avantageusement maintenues en culture pendant une durée d'au moins 70 jours après transfert de gène. De préférence, les cellules transduites sont évaluées à j70 après leur mise en culture, en vue d'une utilisation ultérieure.

Les cellules souches conditionnées par une composition à activité antioxydante peuvent être mises en culture en conditions d'hypoxie.

Les cellules souches sont ainsi conditionnées en vue de leur manipulation génétique, et en particulier par le transfert de gènes dans ces cellules.

Tout type de gène peut être utilisé dès lors qu'il présente par exemple un intérêt thérapeutique, y compris à titre préventif, ou cognitif.

L'invention a donc aussi pour objet une méthode de traitement in vitro de cellules souches, comprenant une étape de conditionnement par une composition à activité antioxydante. La composition à activité antioxydante répond aux définitions données précédemment et est utilisée pour la réalisation de la méthode dans les conditions décrites ci-dessus.

Dans le cadre de l'invention, la composition à activité antioxydante est apportée aux cellules souches dès la mise en culture des cellules et par exemple dès leur décongélation. La composition à activité antioxydante peut ensuite être apportée pendant toute la durée de la culture et en particulier lors de chaque renouvellement du milieu de culture.

Dans un mode de réalisation particulier de l'invention, la composition à activité antioxydante est ajoutée à la culture de cellules de façon telle que la concentration finale en composition à activité antioxydante est comprise entre 0,5 mM et 10 mM, de préférence entre 1 mM et 2 mM.

La concentration finale de N-acétylcystéine dans la culture de cellules souches hématopoiétiques est d'environ 1 mM.

Selon un mode particulier de réalisation de l'invention, l'utilisation ou la méthode décrite est caractérisée en ce que les cellules souches sont des cellules souches primaires.

S'agissant des cellules souches hématopoiétiques, on notera que les cellules souches primaires hématopoiétiques (ou CFU) sont des cellules multipotentes, caractérisées par leur capacité d'auto-renouvellement d'une part, et leur capacité de reconstitution hématopoiétique d'autre part. Elles sont caractérisées par des marqueurs tels que CD34 et CD38, étant CD34 positives et CD38 négatives pour la majorité d'entre elles, ou pour une petite fraction, CD34 négative et AC133 positive.

Les propriétés de ces cellules en font des candidats particulièrement intéressants dans le cadre de protocoles de transfert de gènes, in vitro ou in vivo, mais leur rareté et, de ce fait, les contraintes qui s'imposent à leur prélèvement chez des patients souffrant de déficit hématologique, renforcent la nécessité de les maintenir en culture dans des conditions permettant leur utilisation efficace.

Selon un autre mode de réalisation particulier de l'invention, l'utilisation et la méthode décrites sont caractérisées en ce que les cellules souches telles que celles qui ont été décrites précédemment sont des progéniteurs. Par rapport aux cellules souches primaires, les progéniteurs ont une capacité d'auto-renouvellement beaucoup plus faible, voire nulle, et pour les progéniteurs hématopoiétiques, une capacité à ne restaurer qu'une partie de l'hématopoièse. Les progéniteurs hématopoiétiques, oligopotents, susceptibles d'être traités dans les conditions de l'invention, sont par exemple les progéniteurs lymphocytaires communs (CPL) ou les progéniteurs myéloïdes communs (CMP), ou des progéniteurs érythro/mégacayocytaires (MEPs) ou granulocytes/monocytes/macrophages (GMPs) des cellules présentatrices d'antigènes des cellules dendritiques, notamment des cellules de Langerhans.

Alternativement, l'invention permet avantageusement le conditionnement de cellules souches qui sont des précurseurs et, selon un mode de réalisation particulier de l'invention, des précurseurs hématopoiétiques. Ces cellules sont orientées et n'ont plus de capacité d'auto-renouvellement. Elles peuvent, par prolifération, ou différenciation, conduire aux cellules matures de leur lignée.

Les cellules souches conditionnées selon l'invention peuvent être des cellules pathologiques. C'est notamment le cas des cellules souches hématopoiétiques prélevées chez des patients atteints de l'anémie de Fanconi, dont le conditionnement selon l'invention et la transduction par des vecteurs rétroviraux est décrite ci-après. D'autres pathologies conduisant à une mort cellulaire programmée, ou apoptose, excessive peuvent être concernées.

Les cellules souches peuvent par exemple être prélevées à partir de la moelle des patients ou à partir du sang de cordon, ou encore être mobilisées dans le sang périphérique.

Les cellules souches que l'on veut traiter selon l'invention peuvent à l'inverse être des cellules normales. Ces cellules peuvent cependant provenir d'un patient souffrant d'une pathologie affectant d'autres types cellulaires ou tissulaires, par exemple de patients souffrant de cancer, de maladies héréditaires du métabolisme, de maladies neurodégénératives, ou de déficits immunitaires, ou par exemple du SIDA.

Les inventeurs ont ainsi observé que des cellules souches hématopoiétiques d'une patiente souffrant de cancer du sein, ont pu être mises en culture après conditionnement au moyen de compositions à activité antioxydante dans des conditions améliorant leur viabilité et leur état. Cette amélioration de la viabilité et de l'état des cellules cultivées a permis d'accroître l'efficacité du transfert de gènes, en particulier en utilisant un vecteur rétroviral dérivé de l'oncorétrovirus de Friend.

Les cellules conditionnées par une composition à activité antioxydante répondant à l'une quelconque des définitions données ci-dessus, lesdites cellules étant des cellules souches, et en particulier des cellules souches primaires, ou des cellules souches ayant conduit à des progéniteurs ou à des précurseurs. Il s'agit avantageusement de cellules souches hématopoiétiques, et par exemple, de cellules souches hématopoiétiques CD34+.

Comme indiqué précédemment ces cellules peuvent être pathologiques ou normales.

Selon un mode de réalisation particulièrement préféré de l'invention, la composition à activité antioxydante est constituée par la N-acétylcystéine, dans les conditions de concentration décrites ci-dessus.

Un milieu de culture formulé pour le conditionnement de cellules souches, comprenant une composition ayant une activité antioxydante peut être aussi préparé Le milieu de culture répond, en ce qui concerne la composition à activité antioxydante, aux définitions qui ont été données ci-dessus.

Le milieu de culture peut être préparé à partir de milieux disponibles, plus particulièrement de milieux synthétiques sans sérum. A titre d'exemple, on cite le milieu Cellgro SCGM.

Dans la partie expérimentale qui suit, l'utilisation d'une composition à activité antioxydante constituée par la N-acétylcystéine est décrite dans le cadre de la transduction de cellules souches hématopoiétiques de patients atteints de l'anémie de Fanconi. Elle peut, selon les mêmes modalités, être mise en oeuvre dans d'autres pathologies induisant notamment un déficit médullaire.

Les figures qui suivent illustrent:
Figure 1: constructions de vecteurs utilisées pour la transduction de cellules souches hématopoiétiques d'un patient atteint de l'anémie de Fanconi.
Figures 2 à 5: analyse de l'effet du conditionnement de cellules souches hématopoiétiques par la N-acétylcystéine (NAC) sur le titre cellulaire, de cellules transduites par le vecteur 4B3 portant le gène normal FANCA (figures 2 et 3), de cellules infectées avec C5 (vecteur FOCHA-neo-IRES-CD24) pendant 15 jours (figure 4) et sur la survie des particules rétrovirales (figures 5).

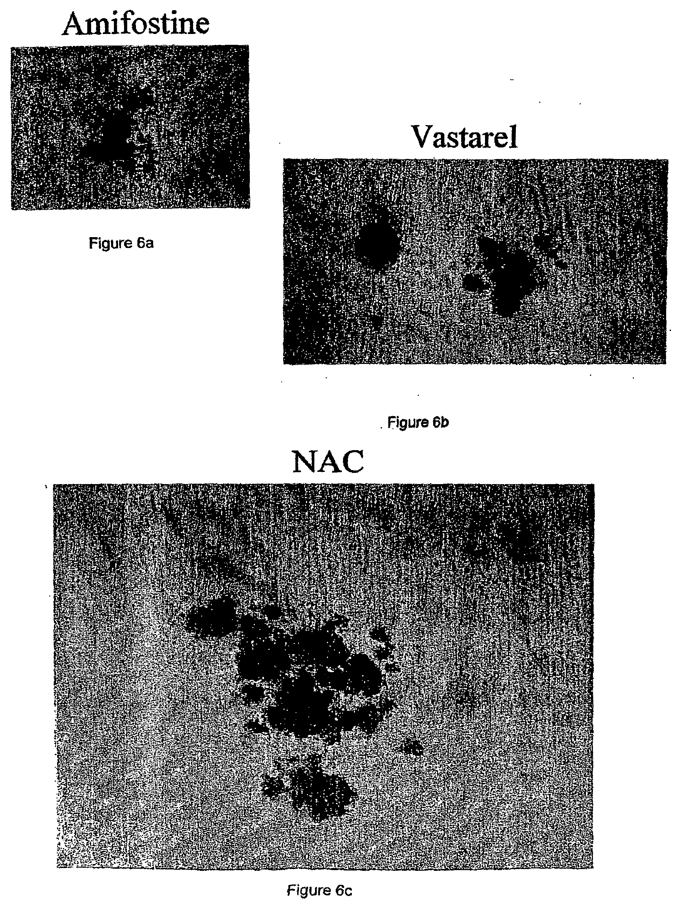

Figure 6: cellules non transduites formant des CFUs, conditionnées avec l'AMIFOSTINE (a), le VASTAREL(b) et la N-acétylcystéine (c).
Figure 7 : observation histologique après cytospin des cellules de la LTC poussant en suspension à j65 (les cellules semi-adhérentes n'ont pas été détachées) ; coloration au May-Grunwald-Giemsa.
Figure 7a) : cellule en prophase de mitose attestant d'une activité mitotique des cellules transduites à un stade très tardif de la culture ;
Figure 7b) : groupe de cellules d'aspect précurseur myeloïde avec un rapport nucléo-cytoplasmique faible et des granulations intra-cytoplasmiques ; à noter une cellule au noyau fragmenté d'aspect apoptotique (cadran gauche sous la ligne médiane).
Figure 8 : culture à long terme de cellules primaires de moelle osseuse non fractionnée de patients FA-A à j70 :
Figure 8a) : cellules en suspension de taille variable (ceci correspond à la figure 7b après cytospin) ;
Figure 8b) : petites cellules réfringentes semi-adhérentes aux cellules mésenchymateuses.
Figure 9 : analyse séquentielle du phénotype des cellules incluses dans la culture à long terme en cytométrie de flux, en comparant cellules témoins non transduites (NI) et cellules génétiquement manipulées (3B5) ;
Figure 9a) : marqueurs de la lignée lymphoide (cellules T) : la moelle est donc initialement constituée d'une très large majorité de cellules lymphocytaires (ce qui est parfaitement anormal) qui disparaissent presque complètement dès huit jours de la LTC ; elles sont remplacées par des cellules myéloides-lignée granuleuse qui s'amplifient dans le puits transduit ;
Figure 9b) : elles sont remplacées par des cellules myéloides-lignée granuleuse qui s'amplifient dans le puits transduit ;
Figure 9c) : ainsi qu'apparaissent des cellules de la lignée érythroide avec une augmentation régulière de ce pourcentage attestant d'une normalisation très significative car l'absence de précurseurs de cette lignée est caractéristique de l'anémie de Fanconi ;
Figure 9d) : présence d'un contigent de la lignée monocyte-macrophage.
Figure 10: j65 de culture à long terme, analyse en microscopie confocale :
Figure 10a) : Marquage CD24+ de cellules transduites avec le vecteur FOCHA-FANCA-IRES-CD24 à j65 de culture. Toutes les cellules observées sont positives et donc transduites ;
Figures b) et c) : la taille et la morphologie des cellules transduites sont polymorphes.
Figure 11 : microscopie confocale : marquage CD34 (Alexa) et visualisation du noyau en iodure de propidium à j70 de la LTC.
Figure 11a) : cellule CD34+ avec un rapport nucléocytoplasmique nettement différent de la majorité des cellules de la LTC ;
Figure 11b) : groupe de cellules avec une seule dont le marquage apparaît significatif (en haut à gauche) ; à noter, discret signal juxta-nucléaire dans la celllule du cadrant inférieur gauche.

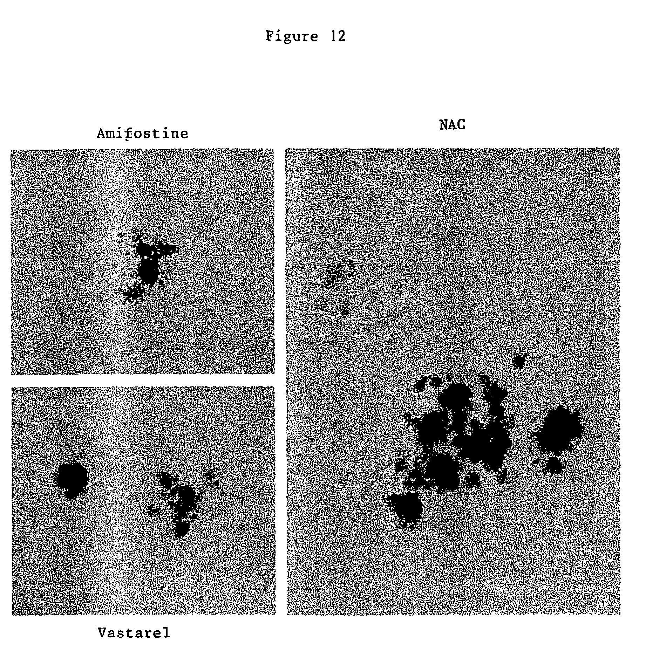

Figure 12 : comparaison de l'effet de plusieurs antioxydants ou composés à effet protecteur sur la capacité clonogénique de cellules primaires de moelle de patients atteints d'anémie de Fanconi, plantées 36 heures après décongélation et mise en présence immédiate des composés. Les cellules non conditionnées ne sont pas représentées, aucune colonie n'étant observée. L'effet tout à fait singulier de la NAC est matérialisé sur cette figure par la différence de taille des colonies obtenues (nombre de cellules radicalement différent). La différence en nombre des colonies n'apparaît pas ; elle est cependant tout aussi significative (deux-trois contre plusieurs dizaines avec la NAC).

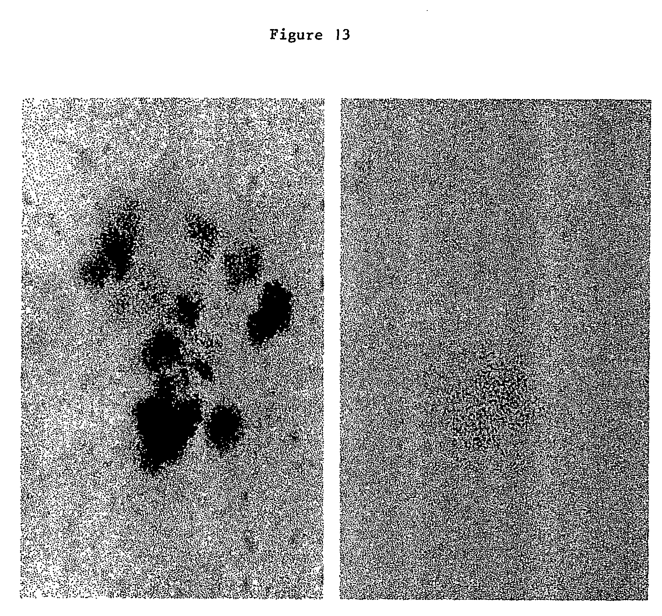

Figure 13: idem à la figure 12 avec indication de l'effet tout à fait singulier de la NAC sur la nature des colonies obtenues : à la fois BFU-E et CFU-GM.

### Exemples

### Problématique

### L'anémie de Fanconi : caractéristiques générales

La maladie de Fanconi, le Xéroderma pigmentosum et l'Ataxie-télangiectasie constituent des maladies représentatives d'un groupe de maladies génétiques héréditaires rares caractérisées par une hypersensibilité aux agents capables d'altérer l'ADN; ainsi qu'une prédisposition marquée au développement de proliférations malignes. Ces maladies ont été regroupées sous le terme plus général de MALADIES HEREDITAIRES DE LA REPARATION DE L'ADN.

### Plusieurs entités génétiques :huit groupes de complémentation

Au moins huit groupes de complémentation distincts ont été identifiés sur la base d'expériences de fusion cellulaire FA-A à FA-E (Strathdee et al, Nat Genet 1992a; 1(3) :196-8; Joenje et al.,Human Genet 97: 280-282 1996). La proportion de patients appartenant à chacun des groupes reste imprécise, mais FAA est très largement majoritaire (70%).

### Localisation de cinq gènes (sur huit) / Pathologie moléculaire

Le premier pas a été franchi lors du clonage du gène *FACC,* localisé en 9q22.3, défectueux dans le groupe C de complémentation (Strathdee et coli., 1992 Nature : 356, 763-767). L'identification des mutations du gène *FAC* a été utilisée pour identifier plus précisément la proportion des patients appartenant à ce groupe (Auerbach et coll. Cancer Genet Cytogenet 51 : 1-12, 1991; Verlander et al., 1994 Am J of Hum Genet 1994 54 : 595-601/ IFAR). Whitney et coll. (1995) ont localisé le gène de l'anémie de Fanconi groupe D (FA-D) sur le bras court du chromosome 3. Plus intéressante encore est la localisation contemporaine de la maladie de Fanconi-groupe de complémentation A sur le chromosome 16q24.3 (Pronk et al.,Nature Genet. 1995 Nov 11 : 338-340). Cette localisation a représenté le premier pas vers le récent clonage positionnel du gène (Lo Ten Foe et al, Nat Genet ; 14 : 320-323 1996).

### Vers une Thérapie Génique de la Maladie de Fanconi

La maladie de Fanconi a actuellement pour seul traitement efficace la greffe de moelle osseuse, dans la mesure où un donneur histocompatible est disponible. L'opportunité de développer une thérapie génique qui consiste à ajouter une copie normale du gène déficient dans les cellules souches hématopoiétiques représente un objectif d'intérêt majeur, accessible à moyen terme. Cette approche est susceptible de s'adresser à une majorité de malades.

Un avantage sélectif in vivo des cellules génétiquement corrigées est attendu. Ce phénomène pourrait permettre une colonisation et une reconstitution sans nécessité de recourir à une myéloablation préalable. La prévention potentielle du syndrome myélodysplasique et de la leucémie devra être évaluée.

En effet, le rétablissement d'une hématopoièse fonctionnelle pourrait prévenir la prolifération de clones précurseurs leucémiques porteurs d'altérations génétiques.

### Situation actuelle du sujet

Des tentatives ont été entreprises antérieurement avec le gène *FANCC* par Liu et coll. (Walsh et al., C Blood 1994 ; 84 : 453-459 ; Liu et al, 1997 Hum Gene Therapy ; 8 : 1715-1730 & Human Gene Therapy ; 1999 10 : 2337-2346). Un essai clinique a été initié au NIH. Tandis que la mobilisation de cellules CD34+ s'est avérée possible, il n'existe aucune évidence à ce jour en faveur de la transduction de cellules hématopoiétiques à durée de vie prolongée. Des limitations d'ordre technologique ont probablement contribué à empêcher un succès clinique.

Ces auteurs ont rapporté des données de correction phénotypique avec le gène *FANCA.* Le vecteur utilisé est le même que pour *FANCC* et les résultats limités à la transduction de cellules clonogénes, uniquement CFU-G ou CFU-GM, sans CFU-E ni CFU-Mix (Fu et al, 1997 Blood ; 90 : 3296-3303).

Les inventeurs se sont intéressés à la transduction des cellules avec une capacité de reconstitution hématopoiétique *in vivo ;* avec le gène *FANCA* et à des démonstrations préalables avec un gène marqueur au moyen du vecteur du laboratoire.

### Stratégie et Matériel et Méthodes

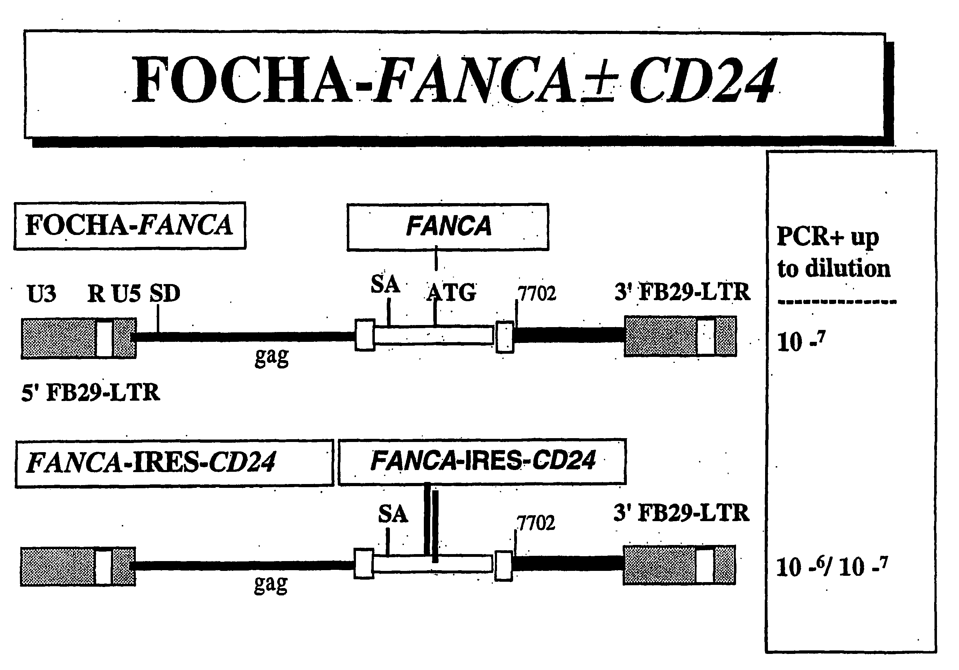

La stratégie proposée repose sur le transfert rétroviral du gène FAA dans les cellules souches hématopoiétiques ; 1°/ construction d'un vecteur rétroviral dérivé du virus de Friend (figure 1), à partir des séquences et des vecteurs décrits dans la demande de brevet WO 95/01447, caractérisé par un tropisme hématopoiétique et une haute infectivité ; 2°/ manipulation génétique de précurseurs CD34+ selon des procédures adaptées à une mise en oeuvre clinique.

### Matériel & méthodes

**1°/** Construction rétrovirale
**2°/** Génération de clones producteurs de hauts titres infectieux
**3°/** Correction phénotypique de lignées lymphoblastoides de patients FA-A et caractérisation des anomalies moléculaires dans un groupe sélectionné de patients FA-A.
**4°/**Transduction de progéniteurs hématopoiétiques CD34+ sélectionnés à partir de sang de cordon ombilical de sujets normaux ; en utilisant des procédures conformes à une utilisation clinique
**5°/** Collection de progéniteurs hématopoiétiques CD34+ chez des patients FA-A. Les progéniteurs sont mis en culture en présence de N-acétylcystéine ajoutée pendant la culture de façon à obtenir une concentration finale de N-acétylcystéine de 1 mM.
**6°/** Transduction de progéniteurs de patients FA-A et évaluation in vitro d'une amélioration fonctionnelle potentielle
**7°/** Reconstitution et/ou persistance à long terme de progéniteurs génétiquement manipulés dans les souris immunodéficientes avec le vecteur WO 95/01447 transmettant soit des gènes marqueurs, soit le gène FANCA. L'évaluation de la transduction et de l'expression est positive à j90.
**8°/** Correction phénotypique de cellules d'animaux *Fanca* -/- transduits avec le gène *FANCA* et reconstitution et/ou persistance à long terme dans des souris syngéniques.
**9°/** Administration in vivo de particules virales dans les cellules souches hématopoiétiques au moyen d'un dispositif original
**10°/** Vérification des conditions et procédures de production de rétrovirus défectifs destinés à un usage clinique : mise en conformité avec la réglementation

### Caractérisation des anomalies moléculaires en vue de la sélection d'un groupe de patients FA-A susceptibles de bénéficier d'une intervention par thérapie génique

Les mutations observées sont de nature très diverse (un total d'une centaine est décrit à ce jour), dispersées sur toute la longueur du gène, dont le cadre de lecture est de 4300 nucléotides environ, répartis en 43 exons sur 80 kb environ (Ianzano et al, Genomics 1997 ; 41 : 309-314). L'étude initiale (Wijker et al. Eur J Hum Genet 1999 : 7: 52-9) a pu mettre en évidence des mutations chez seulement 49% des patients (cohorte de 90 malades). Le spectre de mutation est très hétérogène, celui-ci comprenant des mutations ponctuelles (codon stop), des microdélétions, des insertions, des anomalies d'épissage, et une très grande proportion de délétions intragéniques importantes pouvant comprendre jusqu'à 30 des 43 exons du gène *FANCA.* La méthodologie initialement développée (screening par SSCP, puis séquençage) permettait difficilement de détecter l'ensemble de ce spectre mutationnel. C'est pourquoi, un protocole original d'identification des mutations a été mis au point. Le test analyse (i) les délétions intragéniques : une PCR multiplexe utilisant une combinaison d'amorces est initiée à partir de l'ADN génomique suivie d'une analyse quantitative de la fluorescence par comparaison avec des séquences natives ; et (ii) les mutations après RT-PCR à partir de l'ARN total, détectées par analyse en fluorescence du clivage chimique des mésappariements sur un séquenceur ABI. Cette approche, appliquée sur un échantillon de 26 lignées dérivées de patients FA-A, a mis en évidence au moins une mutation par lignée et 43 allèles mutants sur 52 possibles (Morgan et al. Am J Hum Genet, 1999 ; 65 : 1330-1341). 40% des allèles mutants sont de larges délétions intragéniques qui emportent jusqu'à 31 exons du gène. Ainsi, ce type de mutation pourrait être considéré comme le plus fréquent pour le gène *FANCA.* Plusieurs point communs de délétion sont relevés et il existe une corrélation significative entre le nombre de points de cassure détecté au niveau d'un intron et le nombre de répétitions Alu qu'il contient. Une recombinaison des séquences Alu pourrait ainsi jouer un rôle dans le mécanisme délétionnel.
Des analyses d'éventuelles corrélations phénotype/ génotype sont en cours.

### Correction phénotypique de lignées lymphoblastoides et de fibroblastes primaires de patients FA-A

### Lignées lymphoblastoides

Cette étape nous a permis d'authentifier la transduction et l'amélioration fonctionnelle résultante, de cellules de patients plus faciles à cultiver *in vitro* que des cellules hématopoiétiques primaires. Les jeunes enfants sont ainsi dispensés de prélèvements itératifs de cellules rares et précieuses dont le prélèvement risquerait d'amputer le potentiel hématopoiétique. Une banque de cellules immortalisées a d'ores et déjà été constituée au niveau européen (EUFAR).

Les cellules testées ont des mutations caractérisées du gène *FANCA* ; les cellules témoin comprennent des cellules avec des mutations caractérisées d'un autre gène responsable d'un groupe de complémentation indépendant : le gène *FANCC* et enfin, des cellules lymphoblastoides normales dont la cinétique de croissance sert de référence. Les tests de correction reposent sur une analyse moléculaire des cellules génétiquement manipulées et la démonstration d'une amélioration fonctionnelle attribuable au transfert du gène sain.

Des vecteurs contrôles véhiculant les gènes marqueurs sont utilisés en parallèle avec les vecteurs *FANCA* ± *CD24.* En effet, le cadre de lecture du gène *FANCA* a été taillé à façon afin de s'adapter aux caractéristiques du site de clonage optimisé, pour la transcription et la traduction du vecteur FOCHA *(*Cohen-Haguenauer et al,Hum Gene Ther 1998*).*

L'une des constructions est bicistronique (FOCHA-*FANCA*-IRES-*CD24*) comportant l'ADNc du gène de Fanconi-A *(FANCA)* et une séquence IRES suivie de l'ADNc du gène codant pour l'antigène de surface CD24. Cet épitope permet une identification rapide des cellules après fixation à un anticorps anti-CD24. Cette construction permet de réaliser très rapidement les étapes initiales de validation de l'expression du gène *FANCA* et des conséquences fonctionnelles résultantes. La seconde construction (FOCHA-*FANCA*) ne contient que l'ADNc *FANCA* à des fins de développements cliniques. Les constructions ont été séquencées ; intégralement pour les parties jonctionnelles en 5' et en 3', soit sur les deux tiers distaux ; une analyse en Fluorescence Activated Mismatch Analysis a été entreprise sur la totalité des cadres de lecture. Aucune incompatibilité avec la séquence canonique telle que dessinée, n'a pu être identifiée.

Quelle que soit la construction utilisée (figure 1) (FOCHA*-FANCA* ou FOCHA-*FANCA*-IRES-*CD24*), un échantillon de cellules testées est transduit en parallèle avec le même vecteur transmettant un gène rapporteur. Il s'agit de FOCHA-GFP, véhiculant le gène de la Green Fluorescent Protein (EGFP-1) codant pour la protéine fluorescente verte, utilisée comme marqueur vital dont l'expression dans une cellule donnée peut être suivie dans le temps. L'autre construction utilisée est bicistronique : FOCHA-*NEO*-IRES-*CD24*. Les cellules transduites avec ce vecteur peuvent être à la fois repérées par l'expression de surface de l'antigène CD24 et soumises à une sélection pharmacologique par le G418 (généticine^{R}). Cet outil constitue un excellent élément de référence, lors des expériences réalisées avec le vecteur bicistronique FOCHA-*FANCA*-IRES-*CD24.* Nous disposons de clones producteurs de pseudotypes amphotrope (A/ Récepteur Pit-2) et de pseudotype primate non-humain Gibbon Ape Leukemia Virus (GALV/ Récepteur Pit-1) à partir de lignées productrices d'origine humaine, TE-FLY dérivées de la cellule TE-671 (Cosset et al, 1995). Les surnageants contiennent de hauts titres avec les quatre vecteurs (entre 5x10⁵ entre 5x10⁶ cfu/ml pour les vecteurs *FANCA ;* et entre 5x10⁷ à 10⁸ cfu/ml pour les vecteurs véhiculant des gènes rapporteurs).

Une première étape consiste à authentifier la transduction et étudier sa stabilité. Elle repose sur la conjonction de tests moléculaires, essentiellement PCR et RT-PCR, suivis de traduction in vitro à partir d'ADN ou d'ARN. Une combinaison judicieuse d'amorces de PCR dont les séquences sont données plus loin, spécifiques soit de séquences propres au vecteur rétroviral, soit du cadre de lecture FANCA, permet d'authentifier au niveau moléculaire la présence et l'expression du transgène. En outre, les mutations endogènes affectant la taille ou la stabilité de l'ARN ou de la protéine traduite, sont visualisées. Une seconde étape repose sur le suivi de l'expression de l'antigène CD24. Cet épitope permet une identification rapide des cellules après fixation à un anticorps anti-CD24 et/ ou leur sélection : (i) analyse FACS, (ii) microscopie en fluorescence et confocale ; (iii) ou sélection sur colonnes après fixation (sandwich) sur des billes MACS (Miltenyi-Biotech). Une augmentation hautement significative du nombre des cellules exprimant le CD24 est observée après traitement à la Mitomycine C, puisque les cellules sont pratiquement toutes positives en immunofluorescence.

L'étape cruciale correspond à la démonstration d'une correction phénotypique faisant appel à la normalisation des tests fonctionnels en présence de Mitomycine C (ou de Diépoxybutane) qui établissent le diagnostic de la maladie : **1**°/ Cinétique de croissance des cellules génétiquement manipulées versus cellules témoins : (i) numération cellulaire ; (ii) mortalité cellulaire ; (iii) Cycle cellulaire (FACS), avec réversion du blocage massif en G2, 48 heures après traitement unique par une dose massive de Mitomycine C (100 nM); **2°/** Analyse des cassures chromosomiques, après un traitement prolongé sur plusieurs semaines (6 semaines pour les lignées lymphoblastoides), à doses modérées.

### Fibroblastes primaires (origine médullaire) de patients FA-A

Des prélèvements de moelle osseuse ont été réalisés chez six patients FA. Dans chaque cas, des cultures de cellules adhérentes ont été mises en route et des fibroblastes primaires obtenus et amplifiés (culture ménagée en atmosphère hypoxique). Les cellules primaires ont l'avantage, outre de représenter un matériel humain non modifié par une immortalisation artificielle, d'être potentiellement plus sensibles aux effets de la, correction phénotypique. Des modifications très manifestes de cinétique de croissance cellulaire ont été observées après infection par un clone FOCHA-*FANCA*-IRES-*CD24*. La croissance cellulaire en conditions normoxiques est rétablie. La mise en évidence d'une transduction effective et stable et de la fonctionnalité du transgène est confirmée par une approche en tous points similaire à celle décrite ci-dessus. En particulier, les cellules sont devenues résistantes à de très fortes doses de MMC.

### Cellules hématopoiétiques primaires de patients FA-A

Ceci représente une étape délicate. En effet, les cellules des patients atteints d'Anémie de Fanconi sont caractérisées par un cycle cellulaire altéré et une raréfaction considérable. Les expériences doivent donc être impérativement réalisées sur de faibles nombres de cellules. Cet élément ne fait qu'ajouter à la difficulté de recueil des progéniteurs hématopoiétiques d'une part, et d'autre part à la difficulté technologique de transduction des cellules souches hématopoiétiques. Les données préliminaires des cytaphérèses qui ont été effectuées a *minima* et prudemment, indiquent un rendement très modeste en cellules CD34+ après mobilisation chez les enfants atteints d'Anémie de Fanconi. Il nous est donc apparu indispensable d'avoir recours au prélèvement de moelle osseuse.

Ces cellules ont été maintenues en culture avant toute transduction dans un milieu contenant de la N-acétylcystéine ajoutée dès le début de la mise en culture et pendant la durée de la culture, de sorte que la concentration finale de N-acétylcystéine est de 1 mM. Des expériences préliminaires ont porté sur des échantillons médullaires prélevés chez des enfants aplasiques juste avant le conditionnement pour greffe de moëlle allogénique. Ces données sont encourageantes car, dans ces conditions d'altération extrême de l'hématopoièse, les cultures cellulaires ont pu être maintenues pendant une quinzaine de jours, en hypoxie (5% 02). Même si ces cellules de moelle osseuse ont un potentiel clonogénique initial très faible, voire le plus souvent nul en l'absence d'antioxydant (2-3 CFU pour 100 000 cellules), la transduction de cellules par un gène marqueur (GFP) est mesurable de l'ordre de 2-5% cellules infectées.

L'amélioration du taux de transduction avec des vecteurs contrôles véhiculant des gènes rapporteurs a été permise par l'adaptation des conditions d'oxygénation, l'usage d'antioxydants tels que la N-acétylcystéine, la nature et la concentration des cytokines utilisées.

Les résultats obtenus sur huit patients montrent que : (i) nous avons pu rétablir la culture de cellules médullaires par rapport à des témoins ; l'avantage sélectif des cellules efficacement manipulées est manifeste; (ii) si, en l'absence de manipulation génétique, le nombre de CFUs après 10 jours en culture chute à zéro, nous avons pu obtenir, après transfert du gène normal, non seulement le maintien du nombre de colonies mais encore, une amélioration qualitative en taille et en nature avec apparition de colonies érythroïdes et de colonies mixtes, et même des cellules CD34+ (7%) en fin de culture à j70.

La transduction des cellules de moelle osseuse non fractionnée de patients FA-A a été réalisée comme suit. La procédure a été effectuée sur des cellules cultivées en hypoxie (5% 02) pendant les 28 premiers jours de la culture. A ce stade de la culture (correction fonctionnelle attribuée à l'expression stable du gène correcteur), les cultures ont été secrées de NAC et remises en conditions d'oxygénation normale.

### J0

Les celllules de moelle osseuse non fractionnée de patients FA-A ont été décongelées en milieu SCGM-CellGenix. Elles ont été plantées en plaque de 12 puits ; plusieurs puits ont été plantés. En particulier, les puits témoins non transduits ont été cultivés en parallèle, selon les mêmes procédures. Les cultures ont été réalisées en présence de NAC à la concentration de 1 mM dès la décongélation. Du TPO a été ajouté à raison de 100 ng/ml (pendant les 24 premières heures).

### J1 moins 16 heures

De l'anti-TGFβ a été ajouté aux cellules en culture à une concentration de 10 µg/ml.

A J1 a débuté la transduction. Du surnageant viral préparé directement en milieu SCGM a été ajouté à une MOI de 5-10 particules par cellule.

| | | |
|---|---|---|
| NAC | | 1 mM |
| Anti-TGFβ | 5µg/ml | |
| TPO | | 25 ng/ml |
| LIF | 25 U/ml | |
| SCF/MGDF | | 25 ng/ml |
| FLT-3 | 25 ng/ml | |

A J2 et J3, la procédure a été renouvelée dans les mêmes conditions. Il est important de noter que les cellules n'ont jamais été centrifugées. Aussi, bien qu'elles aient poussé en suspension et n'aient pas été adhérentes, le milieu a simplement et délicatement été prélevé pour les 2/3 et remplacé par le surnageant frais et additionné des cytokines aux concentrations spécifiées à J1.

La NAC était toujours présente dans le milieu de culture à une concentration de 1 mM.

A J4, les cellules ont été placées en conditions de culture à long terme. La culture a été démarrée en suspension. Au fil des jours, des cellules adhérentes de type mésenchymateux sont apparues. Le milieu de culture de base a toujours été le milieu SCGM. La nature des cytokines utilisées et leur concentration ont cependant différé :

| | | |
|---|---|---|
| TPO | | 5 ng/ml |
| LIF | 10 U/ml | |
| SCF/MGDF | | 25 ng/ml |
| IL3 | 5 U/ml | |
| G-CSF | | 10 U/ml |
| Epo | 0,5 U/ml | |
| FLT-3 | | 5 ng/ml |

Ensuite et chaque semaine, le milieu a été renouvelé et les cellules analysées.

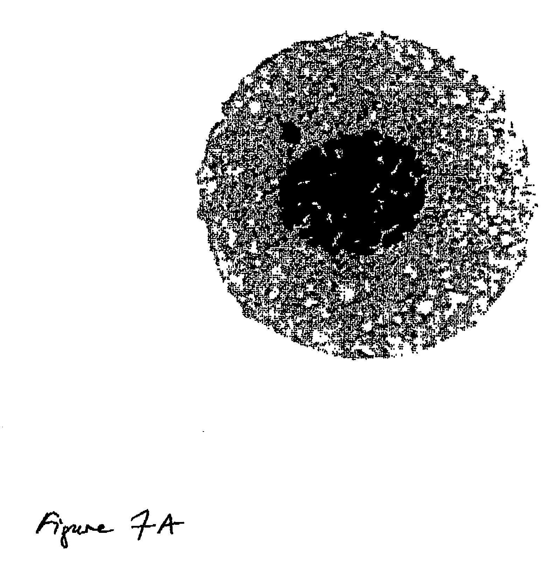

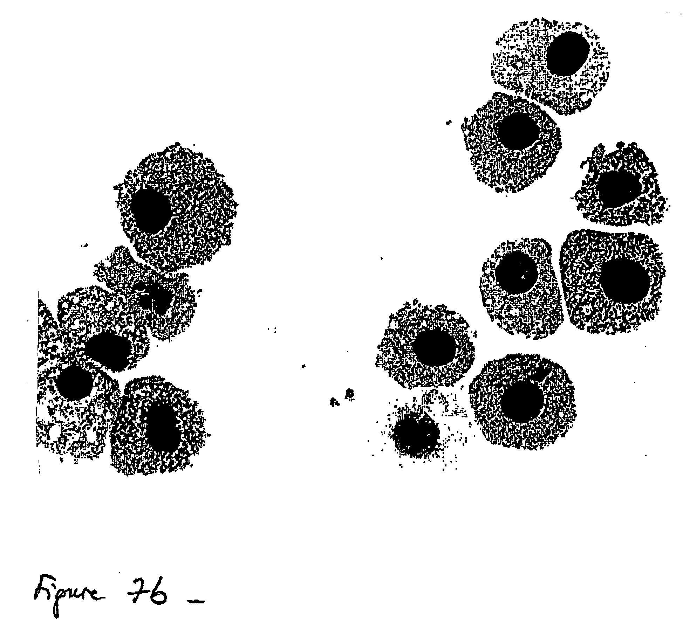

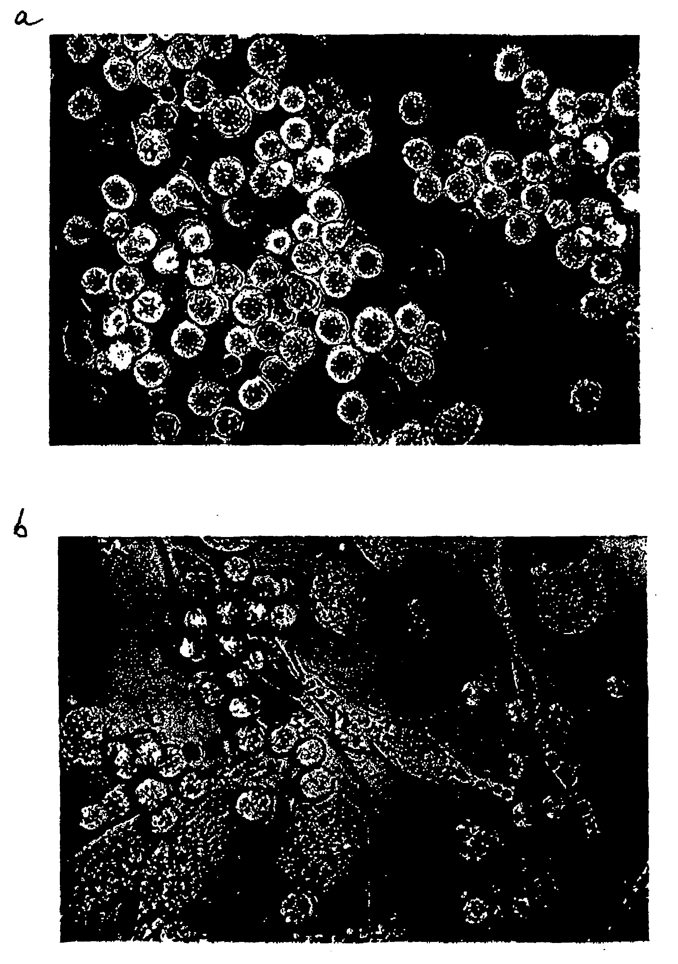

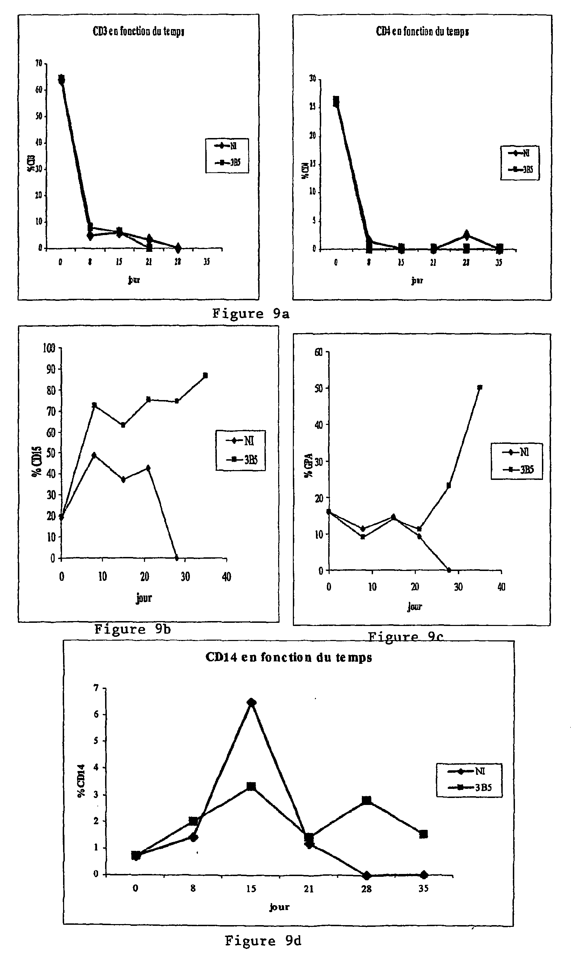

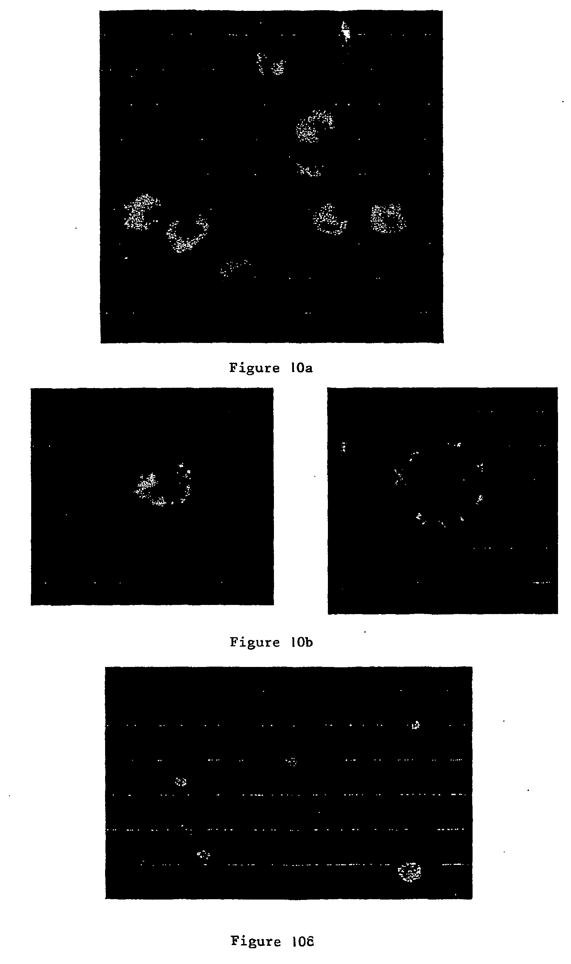

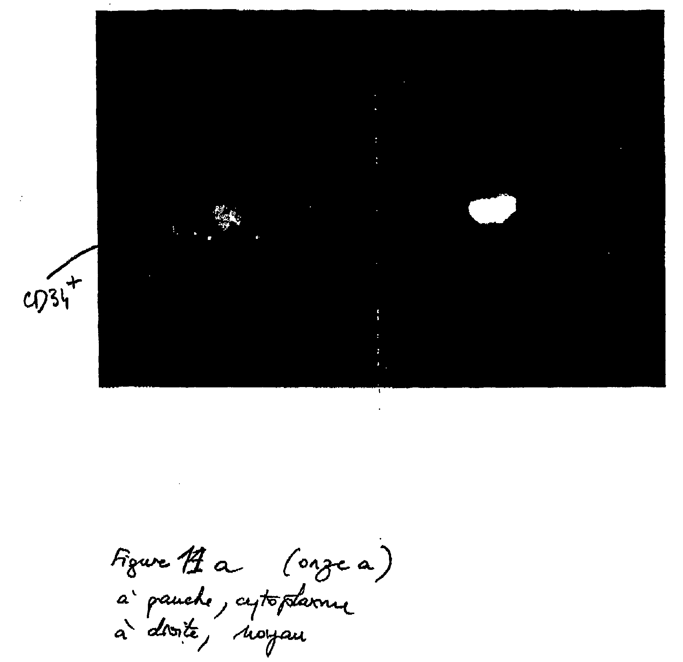

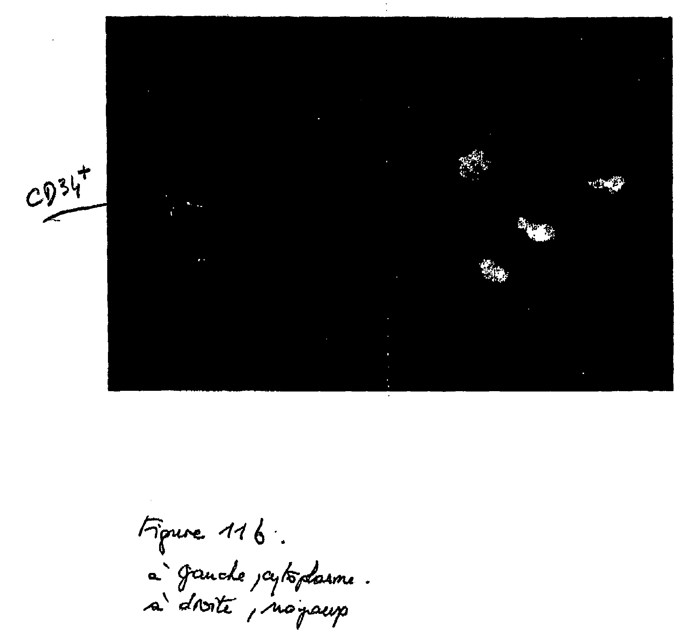

Les résultats obtenus sur la correction génétique des cellules primaires de moelle osseuse non fractionnée de patients FA-A se sont affinés. Nous avons en effet pu mettre en évidence une correction fonctionnelle complète des cellules primaires de moelle de patients Fanconi-A, sur les arguments suivants :
- (1) Dès la troisième semaine et quel que soit l'échantillon, les cultures de cellules non transduites étaient constituées de débris anucléés.
- (2) Culture à long terme sur 70 jours avec images de mitoses (cf figure 7a) et précurseurs myéloides abondants (cf figure 7b). 70% de cellules ne prenaient ni l'annexine ni l'IP à l'analyse au cytomètre de flux : il s'agissait donc de cellules vivantes et de surcroît non apoptotiques, dont le noyau était bien visible avec un rapport nucléocytoplasmique variable.
- (3) Disparition de la fragilité cytoplasmique ; ainsi, la réalisation de cytospin et l'observation entre lame et lamelle étaient devenues possibles.
- (4) Différenciation hématopoiétique harmonieuse au fur et à mesure de la culture à long terme avec des cellules de taille variable (figure 8a) et normalisation du diamètre moyen des cellules qui passe de 5 à 10 microns entre le j1 et j70 (confocal et FACS). Le phénotypage des cellules analysées au cytomètre de flux (suivi séquentiel des marqueurs-FACS) ; figures 9a à 9d) a indiqué en fin de culture à j70, la présence d'un contingent érythroide (30-35 % de cellules Glycophorine A +), mono-macrophagique (3-4 % de CD14+) et beaucoup de granuleux (autour de 60% de CD15+).
- (5) Les cellules présentes dans la culture étaient uniquement des cellules transduites comme en ont attesté les images de microscopie confocale (figures 10a, 10b & 10c). L'analyse FACS a confirmé la présence d'un marquage CD24 sur plus de 65% des cellules. Enfin, les tests moléculaires (PCR et RT-PCR) analysant le gène d'intérêt dans son contexte rétroviral, ont confirmé la transmission et l'intégration du transgène.
- (6) Enfin, de manière tout à fait surprenante et devant l'aspect particulier des cultures à long terme, où était apparu un contingent de superbes petites cellules réfringentes semi-adhérentes aux cellules « mésenchymateuses » (figure 8b), un marquage CD34+ a été retrouvé. Celui-ci a été constaté tant au niveau du microscope confocal (figures 11 a et 11 b) qu'au niveau de analyse par cytométrie de flux. Le contingent CD34+ représentait 7% des cellules analysées à j70 de la culture tandis qu'à j5 aucune cellule CD34 n'était détectée.

Il convient de noter que les procédures expérimentales ont été caractérisées par l'utilisation de concentrations très modestes de cytokines pendant la transduction et de même au cours de la LTC. La présence de NAC dès la décongélation / mise en culture et jusqu'à la correction fonctionnelle des cellules primaires a représenté un élément critique du système. L'objectif poursuivi était de pouvoir permettre aux cellules de se maintenir et de se multiplier sans déclencher une crise proliférative intense et transitoire qui aurait épuisé prématurément la culture. En outre, la réapparition de cellules CD34+ pourrait témoigner de la transduction de cellules avec un potentiel de reconstitution hématopoiétique que la procédure expérimentale serait capable de maintenir tout au long de plus de deux mois de culture in vitro.

Le tableau 1 ci-dessous fournit un résumé de l'effet du conditionnement avec NAC sur les cellules primaires de moelle osseuse de patients FA-A.

**TABLEAU 1**

| **Patient Nombre de cellules j0** | **Standard PO₂** | | **Standard PO₂ + NAC** | | **Hypoxie + NAC** | | **Hypoxie 1 NAC /FICD** | |
|---|---|---|---|---|---|---|---|---|
| **#12** | **NI** | **3,7** | | | | | | |
| **1000 x 10^{E}3** | **FICD** | **98** | | | | | | |
| **#2** | **NI** | **27** | **NI** | **63** | **NI** | **110** | | |
| **1000 x 10^{E}3** | **FICD** | **225** | | | | | | |
| **#2** | **ND** | | **NI** | **60** | **NI** | **104** | **FICD** | **170** |
| **200 x 10^{E}3** | | | | | **j20** | **0** | **j 20** | **70** |

| | | | | | | | | |
|---|---|---|---|---|---|---|---|---|
| - NI = Non transduit - ND = Non fait - FICD = vecteur FOCHA-FANCA-IRES-CD24 | | | | | | | | |

### En parallèle, transduction de progéniteurs hématopoiétiques CD34+ de sujets normaux ; en utilisant des procédures conformes à une utilisation clinique

Cette étape est destinée à démontrer un transfert efficace du gène *FANCA* et une expression résultante à long terme, dans les cellules souches hématopoiétiques humaines, suivant les procédures validées dans notre Laboratoire.

Des expériences de reconstitution à long terme chez l'animal ont été pratiquées sur des souris NOD/SCID, avec les constructions véhiculant les gènes marqueurs soit GFP soit Néo-IRES-CD24, dont l'expression a pu être observée à 6 mois. Ceci constitue le meilleur modèle d'évaluation du comportement des cellules souches de patients explantées, modifiées génétiquement *in vitro,* puis réimplantées. Le vecteur FICD a lui aussi été testé. Transduction et expression ont été vérifiées à j90 sans aucune pression de sélection.

L'efficacité du transfert du gène *FANCA* a été vérifiée sur des cellules de moelle d'animaux Fanca -/-. Un modèle de greffe syngénique chez la souris *fanca-*/*-* est en cours de réalisation. Si la souris ne présente spontanément aucune anomalie de l'hématopoièse, une hypersensibilité est retrouvée après exposition des animaux *in vivo* aux agents toxiques pour les cellules FA. Ainsi, l'administration séquentielle de doses non létales aboutit progressivement à une diminution de tous les paramètres de la numération sanguine. Ce régime de traitement affecte sélectivement le compartiment médullaire sans toucher les autres tissus prolifératifs. Des expériences visant à tester le bénéfice de la transduction de cellules murines pour obtenir une abolition de la symptomatologie fonctionnelle après administration chronique de MMC ont été réalisées.

Des cellules clonogéniques primaires de moelle osseuse de souris déficientes pour le gène *fanca* ont été transduites par un vecteur rétroviral pseudotypé au moyen de la glycoprotéine d'enveloppe VSV-G, et porteur du gène *FANCA* humain. La transduction a été réalisée à raison de 2 infections sur 2 jours avec une MOI de 5. D'une part, 25 % des cellules transduites par le vecteur rétroviral étaient résistantes à 10 nM de MMC. D'autre part, le gène *FANCA* humain était capable de complémenter la déficience génétique des souris.

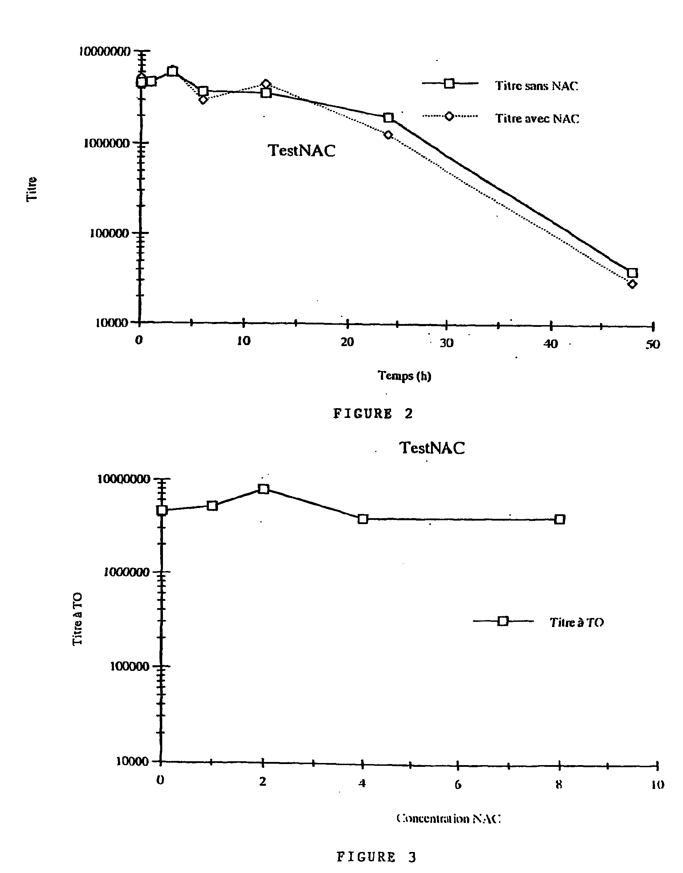

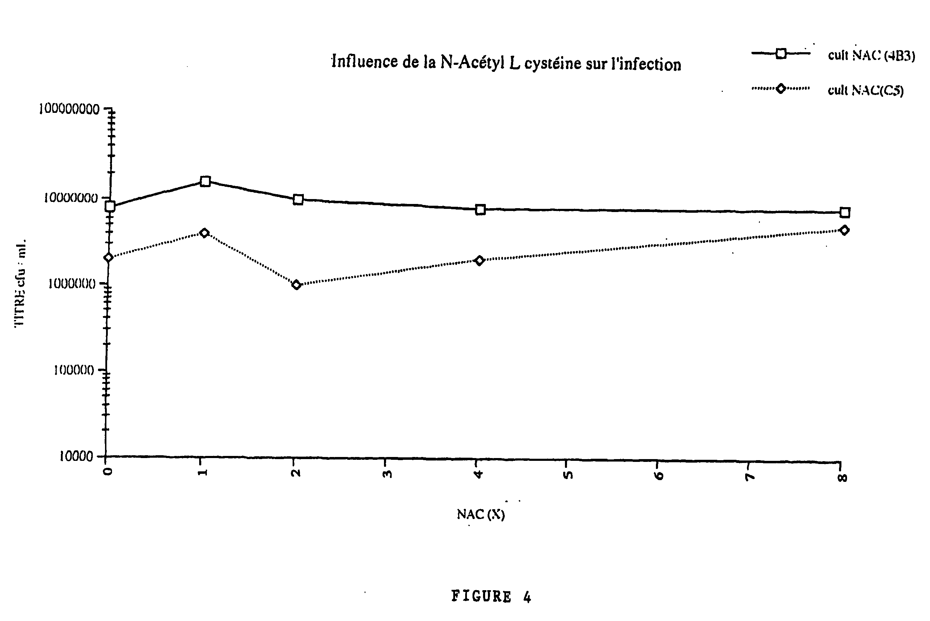

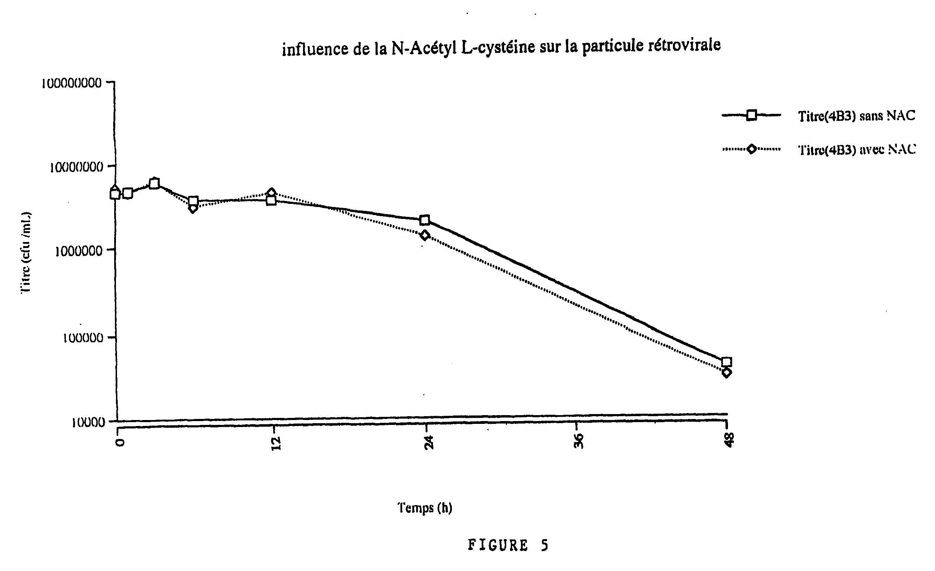

L'absence d'interaction due à la présence de N-acétylcystéine sur la viabilité des particules vecteurs mises en oeuvre dans les expériences de transduction et sur l'infection des cellules cibles par ces particules est présenté sur les courbes constituant les figures 2 à 5. Ainsi, le conditionnement des cellules cibles avec la N-acétylcystéine ne perturbe pas la cinétique des particules vecteurs rétrovirales et n'affecte pas les titres cellulaires des cellules transduites.

Les tableaux qui suivent présentent des résultats de numération des cellules souches hématopoiétiques à différents moments de la culture cellulaire, faisant apparaître l'amélioration de l'état des cellules en présence de N-acétylcystéine, cette amélioration étant observée à la fois pour les cellules souches hématopoiétiques transduites et pour les cellules souches hématopoiétiques non transduites, dès lors qu'elles sont conditionnées par la N-acétylcystéine.

| | **OLIGOS PCR FANCONI (5' → 3')** |
|---|---|
| • RT-PCR (PTT) : | |
| PTT FAA-S1 | CGC TAA TAC GAC TCA CTA TAG GAA CAG ACC ACC ATG TCT CAA AGT GGA CGG CAT |
| PTT FAA-ATG | CGC TAA TAC GAC TCA CTA TAG GAA CAG ACC ACC ATG GCC GAC TCG TGG GTC CCG |
| PTT FAA-S2 | CGC TAA TAC GAC TCA CTA TAG CAG ACC ACC ATG CGC TCC CTC CTC ACA GAC |
| PPT FAA-S3 | CGC TAA TAC GAC TCA CTA TAG GAA CAG ACC ACC ATG TCC CAG GAG CAC TTC CTC |
| PTT FAA-S3N | CGC TAA TAC GAC TCA CTA TAC GAA. CAG ACC ACC ATG GTC AAC GCA CTG ATG GAT |
| PTT FAA-A1 | GTC CCC AGC TGA TGA CAA AT |
| PTT FAA-A2 | AGC AGC TCC CTC TGT CTC TG |
| PTT FAA-ASTOP (A3) | TCA GAVA GAG ATG AGG CTC CTG |
| PTT FAA-S1J | GGA TCC TAA TAC GAC TCA CTA TAG GGA GAC CAC CAT G TCT CAA AGT GGA CGG CAT TGC |
| PTT FAA-ATGJ | GGA TCC TAA TAC GAC TCA CTA TAG GGA GAC CAC CAT G GCC GAC TCG TGG GTC CCG |
| PTT FAA-S2J | GGA TCC TAA TAC GAC TCA CTA TAG GGA GAC CAC CAT G CGC TCC CTG CTC ACA GAC TAC |
| PPT FAA-S3J | GGA TCC TAA TAC GAC TCA CTA TAG GGA GAC CAC CAT G TCC CAG GAG CAC TTC CTC TTT |
| PTT FAA-S3NJ | GGA TCC TAA TAC GAC TCA CTA TAG GGA GAC CAC CAT G GTC AAC GCA CTG ATG GAT TTC |

| • PCR: | |
|---|---|
| ANTI 81 | GAT CTG AAC TTC TCT ATT CTT G |
| NS1 | G4G AAA CTT CTG CTC CCA CG |
| NS2 | TTG ACC TCT GCT CTG GTG TG |
| FW 150 | ACT GGA ACT TCA AGT AAC TCC |
| RV 144 | TTT CAC TGG AAT AAA TCT GCG |
| RV 283 | AGA GAG AGT GAG ACC ACG AAG |
| JB 2195 | CTG GTG GTA TTG TTG AGT CAG G |
| FAA end ORF | GCC TGG AGC TCA AGG GTC AGG G |

**Cellules moelle osseuse avec et sans NAC et/ou Vastarel**

| | **Exp** | **CFUs** | **sans** | | **sans** | | **sans** | | **sans** | |
|---|---|---|---|---|---|---|---|---|---|---|
| | | | Normoxie | | Normoxie | | Normoxie | | Hypoxie | |
| | | | Numération | | Numération | | Numération | | Numération | |
| | | | | | | | | | | |

| | **#1 Javier** | | | | | | | | | |
|---|---|---|---|---|---|---|---|---|---|---|
| | 8/07/99 | | J4 | 12/07/1999 | J11 | 19/07/1999 | J13 | 21/07/1999 | | |
| | 1000x10E3 | Testé | 620 X 10E3 | | NI | 35 x10E3 | NI | 3.7 x10E3 | | |
| | | Aucune observée | J7 | 15/07/1999 | 483 | 80 x 10E3 | 4B3 | 33 x 10E3 | | |
| | | | 360 x 10E3 | | 3B5 | 55 x 10E3 | 3B5 | 98 x 10E3 | | |
| | | | | | | | | | | |

| | **#2 Alex** | | | | | | | | | |
|---|---|---|---|---|---|---|---|---|---|---|
| | 16/09/99 | | J8 | 24/09/1999 | J12 | 2810912000 | | | JB | 24/09/1999 |
| | 1000x10E3 | | NI | 27 x10E3 | NI | 35 x10E3 | | | 3B5 | 225 x 10E3 |
| | | | | | | | | | | |

| | **#3 Alex** | | | | | | | | | |
|---|---|---|---|---|---|---|---|---|---|---|
| | 15/12/99 | | | | | | | | | |
| | 200 x 10E3 | 63 colonies | | | | | | | | |
| | | pour 50x 10E3 | | | | | | | | |
| | | | | | | | | | | |
| | | | | | | | | | | |

| | **#4 Alex** | | | | | | | | | |
|---|---|---|---|---|---|---|---|---|---|---|
| | 18/01/00 | | | | | | | | | |
| | 200 x 10E3 | | | | | | | | | |
| | | | | | | | | | | |
| | | | | | | | | | | |
| | | | | | | | | | | |
| | | | | | | | | | | |
| | | | | | | | | | | |
| | | | | | | | | | | |

| | **#5 Javier** | | | | | | | | | |
|---|---|---|---|---|---|---|---|---|---|---|
| | 4/05/00 | | | | | | | | | |
| | 1000x10E3 | | | | | | | | | |
| | | | | | | | | | | |
| | | | | | | | | | | |

| | **#6 Javier** | | | | | | | | | |
|---|---|---|---|---|---|---|---|---|---|---|
| | 5/06/00 | | | | | | | | | |
| | 400x10E3 | | | | | | | | | |
| | | | | | | | | | | |
| | | | | | | | | | | |
| | | | | | | | | | | |
| | | | | | | | | | | |
| | **Glossaire** | | **NI = non Transduit** | | | | | | | |
| | | | **4B3=FOCHA-GFP** | | | | | | | |
| | | | **3B5 = FOCHA-FANCA** | | | | | | | |
| | | | **Prélèvement : des cellules ont été prélevées pour analyse : Bio Moléculaire / FACS /Immuno-fluorescence** | | | | | | | |
| | | | **Vastarel = Trimétazidine di-chlorhydrate** | | | | | | | |

**Cellules moelle osseuse avec et sans NAC ét/ou Vastarel**

| **Exp** | **avec Nac** | | **avec Nac** | | **avec Nac** | | **avec Nac** | | **avec Nac** | | **avec Nac** | |
|---|---|---|---|---|---|---|---|---|---|---|---|---|
| | Normoxie | | Normoxie | | Normoxie | | Hypoxie | | Hypoxie | | Hypoxie | |
| | Numération | | Numération | | Numération | | Numération | | Numération | | Numération | |
| | | | | | | | | | | | | |

| **#1 Javier** | | | | | | | | | | | | |
|---|---|---|---|---|---|---|---|---|---|---|---|---|
| 8/07/99 | | | | | | | | | | | | |
| 1000x10E3 | | | | | | | | | | | | |
| | | | | | | | | | | | | |
| | | | | | | | | | | | | |
| | | | | | | | | | | | | |

| **#2 Alex** | | | | | | | | | | | | |
|---|---|---|---|---|---|---|---|---|---|---|---|---|
| 16/09/99 | J8 | 24/09/1999 | | | | | J8 | 24/09/1999 | | | | |
| 1000x10E3 | NI | 63x10E3 | | | | | NI | 110 x10E3 | | | | |
| | | | | | | | | | | | | |

| **#3 Alex** | | | | | | | | | | | | |
|---|---|---|---|---|---|---|---|---|---|---|---|---|
| 15/12/99 | J5 | 20/12/1999 | J9 | 24/12/1999 | | | J5 | 20/12/1999 | J9 | 24/12/1999 | | |
| 200 x 10E3 | NI | 88 x10E3 | NI | 104 x10E3 | | | Ni | 80 x10E3 | NI | 176 x10E3 | | |
| | | | mortalité | 31% | | | | | mortalité | 8% | | |
| | CFUs | 8 | CFUs | 1 | | | CFUs | 29 | CFUs | 11 | | |
| | | | | | | | | | | | | |

| **#4 Alex** | | | | | | | | | | | | |
|---|---|---|---|---|---|---|---|---|---|---|---|---|
| 18/01/00 | J6 | 24/01/2000 | J14 | 1/02/2000 | J20 | 7/02/2000 | J6 | 24/01/2000 | J14 | 1/02/2000 | J20 | 7/02/2000 |
| 200 x 10E3 | NI | 91 x10E3 | NI | 28 x10E3 | NI | Néant | NI | 161 x10E3 | NI | 77 x10E3 | NI | 21 x10E3 |
| | | | | | | | 3B5 | 168 x10E3 | 3B5 | 126 x10E3 | 3B5 | 63 x10E3 |
| | | | | | | | | | | | | |
| | CFUs | 19 | | | | | CFUs/NI | 28 | CFUs/NI | 0 | | |
| | | | | | | | CFUs/385 | 75 | CFUs/3B5 | 20 | | |
| | | | | | | | | | | | | |
| | | | | | | | | | | | | |

| **#5 Javier** | | | | | | | J10 | | J16 | | J23 | |
|---|---|---|---|---|---|---|---|---|---|---|---|---|
| 4/05/00 | | | | | | | 3B5 | 83x 10E3 | 3B5 | 42x 10E3 | 3B5 | 15x 10E3 |
| 1000x 10E3 | | | | | | | 4B3 | 80x 10E3 | 4B3 | 64x 10E3 | 4B3 | 23x 10E3 |
| | | | | | | | | | | | | |
| | | | | | | | | | | | | |

| **#6 Javler** | | | | | | | **J8** | | **J16** | | **J21** | **Prélévement** |
|---|---|---|---|---|---|---|---|---|---|---|---|---|
| 5/06/00 | | | | | | | **3B5** | **200x 10E3** | **3B5** | **256x 10E3** | **3B5** | **53x 10E3** |
| 400x10E3 | | | | | | | **4B3** | **180x 10E3** | **4B3** | **280x 10E3** | **ND** | |
| | | | | | | | | | | | | |
| | | | | | | | | | | | | |
| | | | | | | | | | | | | |
| | | | | | | | | | | | | |
| | | | | | | | | | | | | |
| | | | | | | | | | | | | |
| | | | | | | | | | | | | |
| | | | | | | | | | | | | |
| | | | | | | | | | | | | |

**Cellules moelle osseuse avec et sans NAC et/ou Vastarel**

| **Exp** | **avec Nac** | | **avec Nac** | | **avec Nac** | | | | |
|---|---|---|---|---|---|---|---|---|---|
| | **Hypoxie** | | **Hypoxie** | | **Hypoxie** | | | | |
| | **Numération** | | **Numération** | | **Numération** | | | | |
| | | | | | | | | | |

| **#1 Javler** | | | | | | | | | |
|---|---|---|---|---|---|---|---|---|---|
| 8/07/99 | | | | | | | | | |
| 1000x10E3 | | | | | | | | | |
| | | | | | | | | | |
| | | | | | | | | | |
| | | | | | | | | | |

| **#2 Alex** | | | | | | | | | |
|---|---|---|---|---|---|---|---|---|---|
| 16/09/99 | | | | | | | | | |
| 1000x10E3 | | | | | | | | | |
| | | | | | | | | | |

| **#3 Alex** | | | | | | | | | |
|---|---|---|---|---|---|---|---|---|---|
| 15/12/99 | | | | | | | | | |
| 200X10E3 | | | | | | | | | |
| | | | | | | | | | |
| | | | | | | | | | |
| | | | | | | | | | |

| **#4 Alex** | | | | | | | | | |
|---|---|---|---|---|---|---|---|---|---|
| 18/01/00 | | | | | | | | | |
| 200x10E3 | | | | | | | | | |
| | | | | | | | | | |
| | | | | | | | | | |
| | | | | | | | | | |
| | | | | | | | | | |
| | | | | | | | | | |
| | | | | | | | | | |

| **#5 Javler** | | | | | | | | | |
|---|---|---|---|---|---|---|---|---|---|
| 4/05/00 | | | | | | | | | |
| 1000x10E3 | | | | | | | | | |
| | | | | | | | | | |

| **#6 Javler** | **J31** | **Prélèvement** | **J38** | | **J42** | **Prélèvement** | | | |
|---|---|---|---|---|---|---|---|---|---|
| **5/06/00** | **3B5** | **16x 10E3** | **3B5** | **32x 10E3** | **3B5** | **32x 10E3** | | | |
| **400x10E3** | **ND** | | **ND** | | **ND** | | | | |
| | | | | | **J50** | | | | |
| | | | | | **3B5** | **11x10E3** | | | |
| | | | | | | | | | |
| | | | | | | | | | |
| | | | | | | | | | |
| | | | | | | | | | |
| | | | | | | | | | |
| | | | | | | | | | |
| | | | | | | | | | |

**Cellules moelle osseuse avec et sans NAC et/ou Vastarel**

| **Exp** | **Vastarel** | | **Vastarel** | | **Vastarel** | | **Vastarel** | | **Vastarel** | | **Vastarel** | |
|---|---|---|---|---|---|---|---|---|---|---|---|---|
| | Hypoxie | | Hypoxie | | Hypoxie | | Hypoxie | | Hypoxie | | Hypoxie | |
| | Numération | | Numération | | Numération | | Numération | | Numération | | Numération | |
| | | | | | | | | | | | | |

| **#1 Javler** | | | | | | | | | | | | |
|---|---|---|---|---|---|---|---|---|---|---|---|---|
| 8/07/99 | | | | | | | | | | | | |
| 1000x10E3 | | | | | | | | | | | | |
| | | | | | | | | | | | | |
| | | | | | | | | | | | | |
| | | | | | | | | | | | | |

| **#2 Alex** | | | | | | | | | | | | |
|---|---|---|---|---|---|---|---|---|---|---|---|---|
| 16/09/99 | | | | | | | | | | | | |
| 1000x10E3 | | | | | | | | | | | | |
| | | | | | | | | | | | | |

| **#3 Alex** | | | | | | | | | | | | |
|---|---|---|---|---|---|---|---|---|---|---|---|---|
| 15/12/99 | | | | | | | | | | | | |
| 200 x 10E3 | | | | | | | | | | | | |
| | | | | | | | | | | | | |
| | | | | | | | | | | | | |
| | | | | | | | | | | | | |

| **#4 Alex** | | | | | | | | | | | | |
|---|---|---|---|---|---|---|---|---|---|---|---|---|
| 18/01/00 | | | | | | | | | | | | |
| 200x10E3 | | | | | | | | | | | | |
| | | | | | | | | | | | | |
| | | | | | | | | | | | | |
| | | | | | | | | | | | | |
| | | | | | | | | | | | | |
| | | | | | | | | | | | | |
| | | | | | | | | | | | | |

| **#5** Javler | **J10** | | **J16** | | **J23** | | | | | | | |
|---|---|---|---|---|---|---|---|---|---|---|---|---|
| 4/05/00 | 3B5 | 90x 10E3 | 3B5 | 21x 10E3 | 3B5 | Néant | | | | | | |
| 1000x10E3 | 4B3 | 50x 10E3 | 4B3 | Néant | 4B3 | Néant | | | | | | |
| | | | | | | | | | | | | |
| | | | | | | | | | | | | |

| **#6 Javler** | **J8** | | **J16** | | **J21** | **Prélèvement** | **J31** | | **J38** | | **J42** | **Prélèvement** |
|---|---|---|---|---|---|---|---|---|---|---|---|---|
| 5/06/00 | **3B5** | **190x 10E3** | **3B5** | **168x 10E3** | **3B5** | **38x 10E3** | **3B5** | **9x 10E3** | **3B5** | **16x 10E3** | **3B5** | **14x 10E3** |
| 400x10E3 | **4B3** | **230x 10E3** | **4B3** | **96x 10E3** | **ND** | | **ND** | | **ND** | | **ND** | |
| | | | | | | | | | | | **J50** | |
| | | | | | | | | | | | **3B5** | **5x 10E3** |
| | | | | | | | | | | | | |
| | | | | | | | | | | | | |
| | | | | | | | | | | | | |
| | | | | | | | | | | | | |
| | | | | | | | | | | | | |
| | | | | | | | | | | | | |
| | | | | | | | | | | | | |

## Revendications

1. Utilisation d'une composition à activité antioxydante comprenant de la N-acétylcystéine pour le conditionnement in vitro de cellules souches, autres que des cellules souches embryonnaires humaines, pour maintenir lesdites cellules en culture et/ou leur autorenouvellement et/ou leur amplification et/ou leur différenciation et/ou leur prolifération et/ou la régénération de leur potentiel souche.

2. Utilisation selon la revendication 1, dans laquelle le conditionnement des cellules souches est réalisé dans des conditions permettant le transfert de gènes dans ces cellules.

3. Méthode de traitement in vitro de cellules souches autres que des cellules souches embryonnaires humaines, comprenant une étape de conditionnement desdites cellules avec une composition à activité antioxydante comprenant de la N-acétylcystéine.

4. Méthode selon la revendication 3, dans laquelle la composition antioxydante est ajoutée au début de la mise en culture des cellules et optionnellement en cours de culture.

5. Utilisation ou méthode selon l'une quelconque des revendications 1 à 4, dans laquelle la composition à activité antioxydante est apportée à une culture de cellules souches hématopoïétiques, à une concentration finale dans la culture de 0,5 à 10 mM.

6. Utilisation ou méthode selon l'une quelconque des revendications 1 à 5, dans laquelle les cellules souches sont cellules souches primaires.

7. Utilisation ou méthode selon l'une quelconque des revendications 1 à 5, dans laquelle les cellules sont des progéniteurs issus de cellules souches.

8. Utilisation ou méthode selon l'une quelconque des revendications 1 à 5, dans laquelle les cellules sont des précurseurs issus de cellules souches.

9. Utilisation ou méthode selon l'une quelconque des revendications 1 à 5, dans laquelle les cellules souches sont des cellules pathologiques.

10. Utilisation ou méthode selon l'une quelconque des revendications 1 à 5, dans laquelle les cellules souches sont des cellules normales.

11. Utilisation ou méthode selon l'une quelconque des revendications 1 à 5, dans laquelle les cellules souches sont des cellules prélevées chez un patient atteint d'un cancer, de maladies héréditaires du métabolisme, de maladies neurodégénératives, de déficits immunitaires, ou par exemple du SIDA.

12. Utilisation ou méthode selon l'une quelconque des revendications 1 à 11, dans laquelle les cellules souches conditionnées avec une composition à activité antioxydante sont transformées par un vecteur portant une séquence nucléotidique d'intérét.

13. Utilisation ou méthode selon l'une quelconque des revendications 1 à 12, dans laquelle les cellules souches conditionnées avec une composition à activité antioxydante sont des cellules souches choisies parmi les cellules souches hématopoïétiques, les hémangioblastes, les cellules souches du système nerveux central, les cellules souches mésenchymateuses, les cellules souches musculaires, les cellules souches dérivées de la moelle osseuse, les cellules souches musculaires ou hépatiques dérivées de la moelle osseuse.

## Claims

1. Use of an antioxidant composition comprising N-acetylcysteine for in vitro conditioning of stem cells, except human embryonic stem cells, for maintaining said stem cells in culture and/or their auto turn over and/or their amplification and/or their differentiation, and/or their proliferation and/or the regeneration of their stem potential.

2. The use according to claim 1, wherein the conditioning of stem cells is made under conditions allowing the transfer of genes into said cells.

3. An, in vitro therapy method of stem cells, except human embryonic stem cells, comprising a step of conditioning said cells with an antioxidant composition comprising N-acetylcysteine.

4. The method according to claim 3, wherein the antioxidant composition is added at the beginning of the culture of the cells and optionally during the culture.

5. The use or method according to any of claims 1 to 4, wherein the antioxidant composition is added in the culture of hematopoietic stem cells at a final concentration of 0,5 to 10 mM.

6. The use or method according to any of claims 1 to 5, wherein the stem cells are primary stem cells.

7. The use or method according to any of claims 1 to 5, wherein the cells are progenitor cells coming from stem cells.

8. The use or method according to any of claims 1 to 5, wherein the cells are precursor coming from stem cells.

9. The use or method according to any of claims 1 to 5, wherein the stem cells are pathologic cells.

10. The use or method according to any of claims 1 to 5, wherein the stem cells are normal cells.

11. The use or method according to any of claims 1 to 5, wherein stem cells are cells removed from a patient affected by a cancer, metabolic hereditary diseases, neurodegenerative diseases, immune deficiency, or AIDS for instance.

12. The use or method according to any of claims 1 to 11, wherein the stem cells conditioned with an antioxidant composition are transformed by a vector bearing a nucleotidic sequence of interest.

13. The use or method according to any of claims 1 to 12, wherein the stem cells conditioned with an antioxidant composition are stem cells chosen among hematopoietic stem cells, hemangioblasts, central nervous system stem cells, mesenchymal stem cells, muscular stem cells, bone marrow stem cells, muscular or hepatic bone marrow stem cells.

## Patentansprüche

1. Verwendung einer N-Acetylcystein enthaltenden Zusammensetzung mit antioxidativer Aktivität zur In-Vitro-Aufbereitung von Stammzellen mit Ausnahme von menschlichen embryonalen Stammzellen, zum Halten dieser Zellen in Zellkultur und/oder ihrer Selbsterneuerung und/oder ihrer Amplifikation und/oder Differenzierung und/oder ihrer Proliferation und/oder der Regenerierung ihres Stammzellenpotentials.

2. Verwendung gemäss Anspruch 1, wobei die Aufbereitung der Stammzellen unter Bedingen durchgeführt wird, die den Gentransfer in diese Zellen ermöglichen.

3. Methode zur In-Vitro-Behandlung von Stammzellen mit Ausnahme von menschlichen embryonalen Stammzellen, die eine Aufbereitungsstufe besagter Zellen mit einer N-Acetylcystein enthaltenden Zusammensetzung mit antioxidativer Aktivität beinhaltet.

4. Methode gemäss Anspruch 3, bei der die antioxidative Zusammensetzung zu Beginn der Zellkultur oder wahlweise während der Kultivierung der Zellen beigefügt wird.

5. Verwendung oder Methode gemäss einem der Ansprüche 1 bis 4, bei der die Zusammensetzung mit antioxidativer Aktivität einer blutbildenden Stammzellenkultur mit einer Endkonzentration in der Kultur von 0,5 bis 10 mM beigefügt wird.

6. Verwendung oder Methode gemäss einem der Ansprüche 1 bis 5, in der die Stammzellen primäre Stammzellen sind.

7. Verwendung oder Methode gemäss einem der Ansprüche 1 bis 5, in der die Zellen aus Stammzellen stammende Progenitoren sind.

8. Verwendung oder Methode gemäss einem der Ansprüche 1 bis 5, in der die Zellen aus Stammzellen stammende Vorläufer sind.

9. Verwendung oder Methode gemäss einem der Ansprüche 1 bis 5, in der die Stammzellen pathologische Zellen sind.

10. Verwendung oder Methode gemäss einem der Ansprüche 1 bis 5, in der die Stammzellen normale Zellen sind.

11. Verwendung oder Methode gemäss einem der Ansprüche 1 bis 5, bei der die Stammzellen Zellen sind, die einem Patienten mit Krebs, erblichen Stoffwechselkrankheiten, neurodegenerativen Krankheiten, an Immunschwäche, oder zum Beispiel an Aids erkrankten Patienten entnommen wurden.

12. Verwendung oder Methode gemäss einem der Ansprüche 1 bis 11, bei der die mit einer Zusammensetzung mit antioxidierender Aktivität aufbereiteten Stammzellen durch einen Vektor mit einer Nukleotidsequenz von Interesse verändert werden.

13. Verwendung oder Methode gemäss einem der Ansprüche 1 bis 12, bei der für die mit einer Zusammensetzung mit antioxidierender Aktivität aufbereiteten Stammzellen folgende Stammzellen gewählt werden: hämotopoetische Stammzellen, Hämangioblasten, Stammzellen des zentralen Nervensystems, mesenchymale Stammzellen, muskuläre Stammzellen, aus Knochenmark abgeleitete Stammzellen, aus Knochenmark abgeleitete muskuläre oder hepatische Stammzellen.
